# EUROPEAN PATENT SPECIFICATION

(11) **EP 1 772 121 B1**
(45) Date of publication and mention of the grant of the patent: **24.11.2010**
(21) Application number: 06255131.2
(22) Date of filing: 04.10.2006
(51) Int. Cl.: A61F 2/84, A61M 25/01

(54) **Catheter for treating an intimal dissection after stent implantation**
Katheter zur Behandlung von intimaler Dissektion nach der Anordnung eines Stents
Cathéter pour le traitement de dissection intimale suite à la mise en place d'un stent

(30) Priority: 05.10.2005 US 243647
(43) Date of publication of application: 11.04.2007
(73) Proprietor: Cordis Corporation, Miami Lakes, FL 33014 (US)
(72) Inventor: Fischell, David R., Fair Haven, NJ 07704 (US); Burgermeister, Robert, Bridgewater, NJ 08807 (US)
(74) Representative: Fisher, Adrian John

(56) References cited:
- EP-A1- 1 366 731
- WO-A-00/12166
- WO-A-99/13935
- US-A- 5 357 978
- US-A- 5 738 667
- US-A- 6 159 195
- US-A- 20050 059 991
- US-A1- 2005 101 968
- US-B1- 6 537 294

## Description

### FIELD OF USE

This invention is in the field of catheters used to treat intimal dissections which result from the placement of a stent into an artery, such as a coronary artery.

### BACKGROUND OF THE INVENTION

In US patent application serial number 09/444,104, now U. S. Patent No. 6,375,660, a stent delivery catheter is described that has a fixed guidewire at its distal end. Although this system for delivering a stent into a vessel of the human body has the advantage of providing an extremely small outside diameter for easy insertion through even the narrowest of arterial stenoses, it does not provide for those few cases when an intimal dissection occurs after stent implantation. Specifically, if a separate guidewire is used during stent implantation, it can be kept in place after the stent delivery catheter is removed, thus allowing insertion of a second stent delivery system over the guidewire to repair any intimal dissection. However, if the guidewire is fixed to the stent delivery catheter, it will be removed with the stent delivery catheter after the stent has been delivered. Without a guidewire through that portion of the artery where an intimal dissection has occurred, it can be extremely difficult to place a second stent delivery catheter to deliver a second stent to repair such an intimal dissection.

EP-A-1 366 731 discloses a rescue catheter that is designed to be placed over a fixed wire stent delivery catheter after angiography reveals that an intimal dissection has occurred typically as an edge dissection either just proximal or just distal to the stent.

The features known in combination from EP-A-1 366 731 are set out in the preamble of the accompanying claim 1.

There is also disclosed in EP-A-1 366 731 a method for using such a catheter to repair an intimal dissection. It should be understood that an intimal dissection that is in close proximity to the edge of an implanted stent is called an "edge dissection".

As previously stated, US patent application serial number 09/444,104 (now US 6,375,660) describes a fixed guidewire stent delivery catheter with an extremely small outside diameter which can be used to deliver a stent through a very tight arterial stenosis. After the stent on the stent delivery catheter has been delivered into the arterial wall, the balloon is deflated and the fixed guidewire stent delivery catheter would be left in place. Contrast medium would then be delivered to the site of the dilated stenosis to indicate if there is any arterial wall dissection. Such an intimal dissection would typically occur near the proximal or distal edge of the stent.

In most cases there will be no edge dissection, the stent delivery catheter would be removed from the body and the stent implantation would be considered to be successfully completed.

According to the method of EP-A-1366731, if however, the angiography with the contrast medium indicates that an edge dissection has occurred, then the fixed guidewire stent delivery catheter would be left in place with its balloon deflated. The Luer fitting at the proximal end of the stent delivery catheter would then be cut off and a rescue in the form of a rescue catheter would be advanced over the stent delivery catheter. The rescue catheter would then be advanced until its distal end extended distally beyond the site of the edge dissection. The fixed wire stent delivery catheter would then be pulled out of the body through the rescue catheter and a conventional guidewire would be inserted through the rescue catheter. The rescue catheter would then be removed leaving the guidewire in place. A conventional stent delivery catheter would then be used to deliver a second stent to the site of the intimal dissection at the edge of the first implanted stent, thus repairing the dissection.

### SUMMARY OF THE INVENTION

According to the present invention there is provided a rapid exchange rescue catheter that is delivered in a manner similar to a rapid exchange or monorail type angioplasty catheter, except the guiding device is the fixed wire stent delivery system. To be most effective and not require cutting of the fixed wire stent delivery system, the rapid exchange rescue catheter is designed to "snap onto" the shaft of the fixed wire stent delivery system where a guidewire tube is attached to the snap on mechanism and can then be advanced over the fixed wire stent delivery system until the distal end of the guidewire tube is past the intimal dissection in the artery. A guidewire is then advanced through the guidewire tube until it is past the dissection allowing a normal stent procedure to follow.

Documents US 5738667 and US 2005/0059991 describe respectively an exchange member and a guide member comprising snap-on sections to snap on delivery systems or PTCA catheters, but not at the distal end of guidewire tubes.

According to the present invention, a rescue catheter is designed to be placed over a fixed guidewire stent delivery system if angiography indicates that an intimal dissection has occurred, the rescue catheter being designed for placement of a conventional guidewire through it so that a second stent delivery system can be advanced over the guidewire to deliver a stent to repair the intimal dissection.

The rescue catheter treats an intimal dissection created by the delivery of a stent from a fixed guidewire stent delivery system.

Still further, there is provided a rapid exchange rescue catheter whose distal section is designed to snap onto and be guided by the shaft of a fixed wire stent delivery system.

Thus, there is provided a rescue catheter of the type defined in the accompanying claim 1. Preferred aspects are set out in the dependent claims.

### BRIEF DESCRIPTION OF THE DRAWINGS

FIG. 1 is a side view of a prior art fixed guidewire stent delivery system shown with the stent mounted onto the balloon.
FIG. 2 is a longitudinal cross section of the fixed guidewire stent delivery system with the stent shown delivered into an arterial stenosis, the balloon is deflated an a distal intimal dissection has occurred.
FIG. 3 is a side view of the rescue catheter that is adapted to pass through an intimal dissection.
FIG. 4 is a longitudinal cross section of a distal portion of the rescue catheter at section 4-4 of FIG. 3.
FIG. 5 shows the rescue catheter placed over the fixed guidewire stent delivery system and through an intimal dissection.
FIG. 6 shows the rescue catheter in place with the stent delivery system removed and a conventional guidewire placed through the rescue catheter with its distal end placed beyond the intimal dissection.
FIG. 7 shows the guidewire in place and the rescue catheter removed.
FIG. 8 shows an exemplary rescue catheter having an open distal end.
FIGs. 9A and 9B illustrate a first snap-on embodiment of the rescue catheter of the present invention.
FIGs. 10A and 10B illustrate a second snap-on embodiment of the rescue catheter of the present invention having a tapered distal snap-on section for improved slideability.

### DETAILED DESCRIPTION OF THE INVENTION

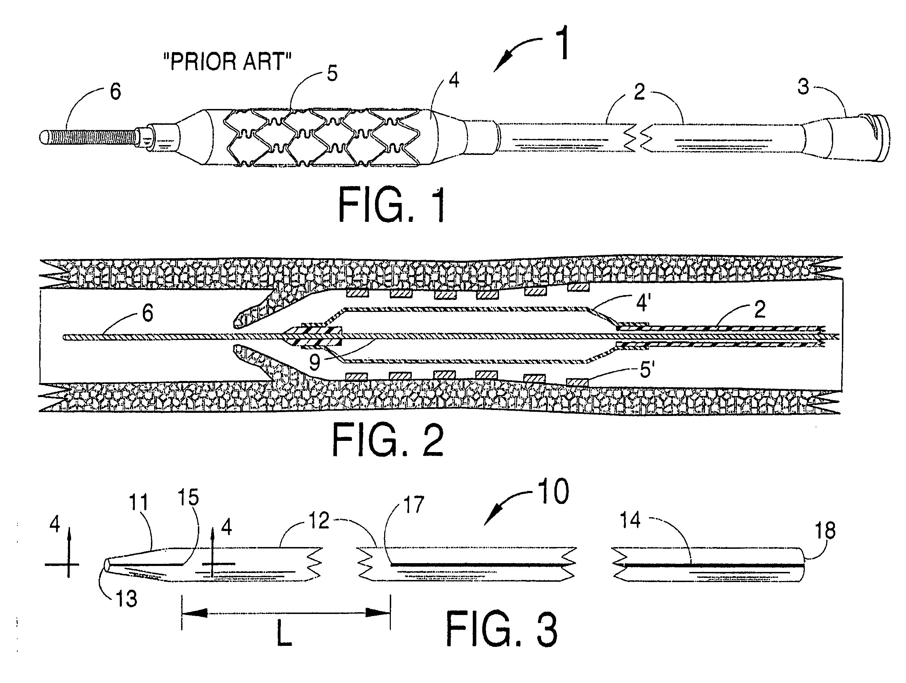

FIG. 1 is a side view of a prior art stent delivery system 1 having a fixed guidewire 6 attached to the distal end of a balloon 4 onto which a stent 5 has been mounted. A shaft 2 which extends for most of the length of the stent delivery system 1 is attached at its distal end to the balloon 4 and at its proximal end to a Luer fitting 3. (This type of stent delivery system is described in detail in US patent number 6,375,660, referred to above). If angiography indicates that there has been an intimal dissection after the stent 5 is placed into an arterial stenosis, the Luer fitting 3 is cut off from the shaft 2 so that a rescue catheter can be advanced over the stent delivery system 1.

FIG. 2 illustrates the delivered stent 5' deployed into an artery with a deflated balloon 4' attached at its distal end to the guidewire 6 and at its proximal end to the shaft 2. A core wire 9 attached to the fixed guidewire 6 can extend to the proximal end of the stent delivery system 1.

FIG. 3 is a side view of a rescue catheter 10 having a shaft 12 that extends for most of its length, an interior lumen 18 and a distal cone 11 located at a distal portion of the catheter 10. The shaft 12 has an elongated slit 14 whose distal end 17 is situated between 0.5 and 20 cm from the proximal end of the cone 11. The cone 11 has a hole at its distal end and at least one slit 15 that extends to and possibly beyond the distal end of the shaft 12. The length of the cone 11 should be less than 10 cm and optimally approximately one cm. The length of the cylindrical shaft 12 should be at least 100 cm and optimally longer than 125 cm. The inside diameter of the shaft 12 should be just slightly larger than the maximum outside diameter of the stent delivery system 1.

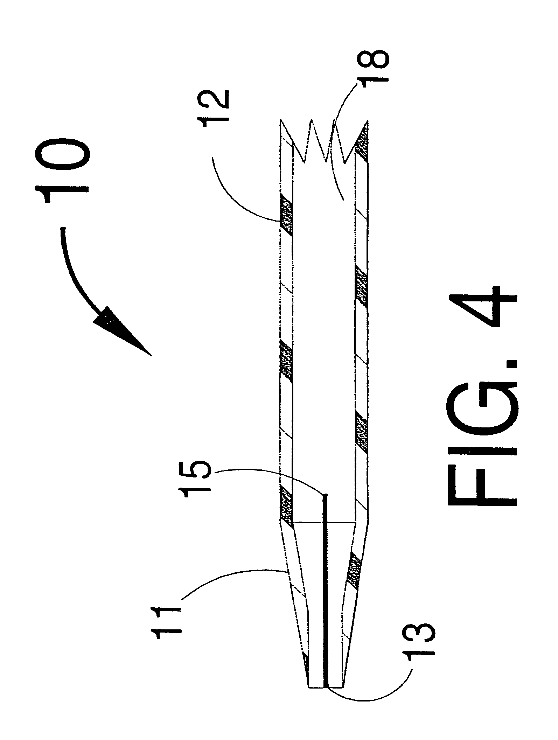

FIG. 4 is a longitudinal cross section of a distal portion of the catheter 10 showing the distal cone 11, the shaft 12, the distal opening 13, a slit 15 and the lumen 18. Although the slit 15 is shown extending into the shaft 12, it is envisioned that its proximal end could remain within the cone 11 or it could extend for as much as a centimeter into the shaft 12. Furthermore, it should be understood that there could be as many as four slits 15 around the circumference of the cone 11 or as few as one.

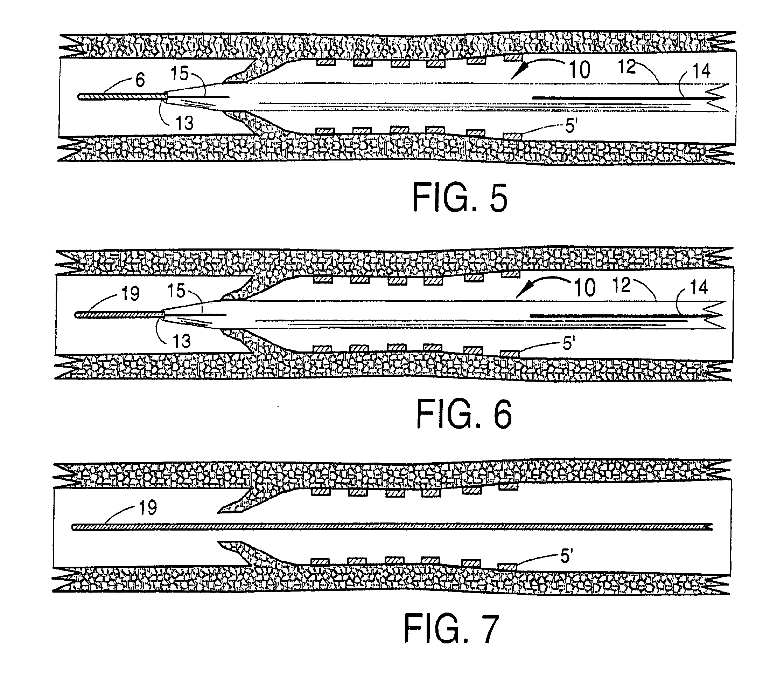

FIGS. 5, 6 and 7 illustrate how the catheter 10 would be used to place a conventional guidewire through an intimal dissection that extends beyond the edge of a stent. FIG. 5 shows the catheter 10 inside an artery into which the stent 5' has been deployed. If there is an intimal dissection 8 extending beyond the edge of the stent 5', it can be detected by the interventional cardiologist using contrast medium. After an intimal dissection is detected, the Luer fitting 3 is cut off from the shaft 2 of the stent delivery system 1. The interventional cardiologist would then open the cone 11 and place it over the cut off proximal end of the shaft 2 of the stent delivery system 1. The interventional cardiologist would then advance the catheter 10 over the shaft 2 until the proximal end of the shaft 2 could be pulled out of the slit 14. While holding the proximal end of the shaft 2 in one hand, the interventional cardiologist would, with his other hand, advance the catheter 10 over the stent delivery system 1 until the opening 13 of the cone 11 was advanced over the guidewire 6 and past beyond the intimal dissection 8. Because of its conical shape, and because the inside diameter of the end hole 13 would be approximately the same as the outside diameter of the guidewire 6, the distal end of the catheter 10 should readily pass through the dissection 8. Since it is expected that the diameter of the guidewire 6 would be 0.36 mm ( 0.014 inches), an optimal diameter for the opening 13 of the cone 11 would be approximately 0.36 mm (0.014 inches).

After the catheter 10 is positioned as shown in FIG. 5, the stent delivery system 1 is pulled back out of the catheter 10 and out of the patient's body. A conventional guidewire 19 is then inserted through the catheter 10 until its distal end lies distal to the intimal dissection 8. This condition is shown in FIG. 6. The catheter 10 is then removed from the patient's body and the guidewire 19 remains in place as shown in FIG. 7. Because of the slit 14 in the side of the shaft 12 of the rescue catheter 10, a conventional length of approximately 135 cm could be used for the guidewire 19. An alternative method would be to use a guidewire 19 that is somewhat more than twice as long as a conventional guidewire.

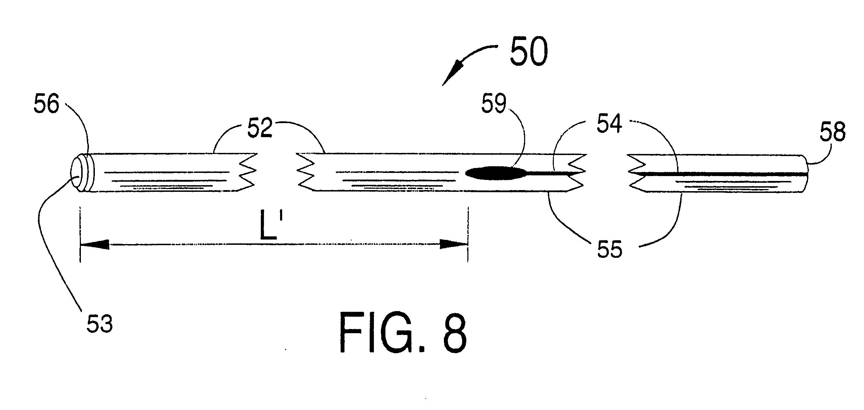

FIG. 8 shows an exemplary rescue catheter 50, known from EP-A-1 366 731, having an open distal end 56 and central lumen 53. The catheter 50 has a distal cylindrical section 52 of length L' and a proximal section 55 with a slit 54 that extends from the proximal end 58 of the catheter 50 to a widened opening 59 at the distal end of the slit 54 which is the proximal end of the distal section 52. The length L' is typically between 0.5 and 20 centimeters.

Similar to the rescue catheter 10 of FIGs. 4 through 7, the rescue catheter 50 can be advanced over the cut off proximal end of the stent delivery system 1. The rescue catheter 50 can either be advanced concentrically over the stent delivery system 1 in an "over-the-wire" type delivery or the rescue catheter 50 can be used in a rapid exchange method where only the distal section 52 is advanced concentrically over the stent delivery system 1. Thus the shaft of the stent delivery system 1 would lie inside the rescue catheter 50 only between the distal end 56 and the opening 59. Ideally as the rescue catheter 50 is advanced, more and more of the shaft of the stent delivery system 1 can be pressed inside of the proximal section 55 through the slit 54. Once the rescue catheter 50 has been advanced distally to the desired location, the stent delivery system 1 can be retracted through the lumen 53 of the rescue catheter 50. A guidewire can then be advanced through the lumen 53 to provide guidance for additional recannalization devices.

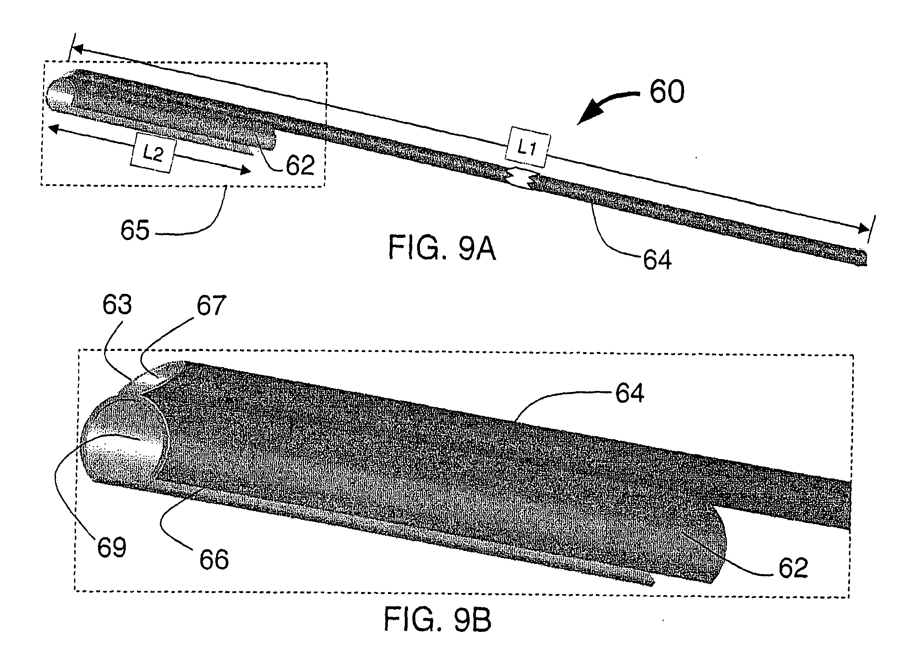

FIG. 9A illustrates a first snap-on embodiment of the present invention rescue catheter 60 having a guidewire tube 64 of length L1 and distal end 65 with slit snap-on section 62 having length L2. L 1 must be sufficiently long so that the distal end of the catheter 60 can reach the desired point in the human vascular system and is typically between 1 and 2 meters. L2 is typically between 0.5 and 20 centimeters.

FIG. 9B shows an enlargement of the distal end 65 of the rescue catheter 60 of FIG. 9A. The distal end 65 includes the slit snap-on section 62 with lumen 69 and slit opening 66. Also shown in FIG. 9B is the lumen 67 and tapered distal end 63 of the guidewire tube 64. The tapered distal end 63 facilitates penetration through a vascular obstruction such as an intimal dissection. Unlike the rescue catheters 10 and 50 of FIGs. 3 and 8, the rescue catheter 60 does not require that the fitting at the proximal end of the stent delivery system 1 be cut off. The slit snap-on section 62 is designed to be snapped onto the shaft 2 of the stent delivery system 1 of FIGs. 1 and 2. This is done by pressing the shaft 2 through the slit 66 until the shaft 2 snaps into the lumen 69 of the slit snap-on section 62. The rescue catheter can be advanced over the shaft 2 and beyond until the distal end 63 of the guidewire tube 64 lies beyond the vascular obstruction. A guidewire can than be advanced through the lumen 67 of the guidewire tube 64 until the distal end of the guidewire lies beyond the vascular obstruction. The rescue catheter 60 and stent delivery system 1 can then be removed from the body and the guidewire can be used to perform additional procedures such as stent delivery, to recannalize the vessel.

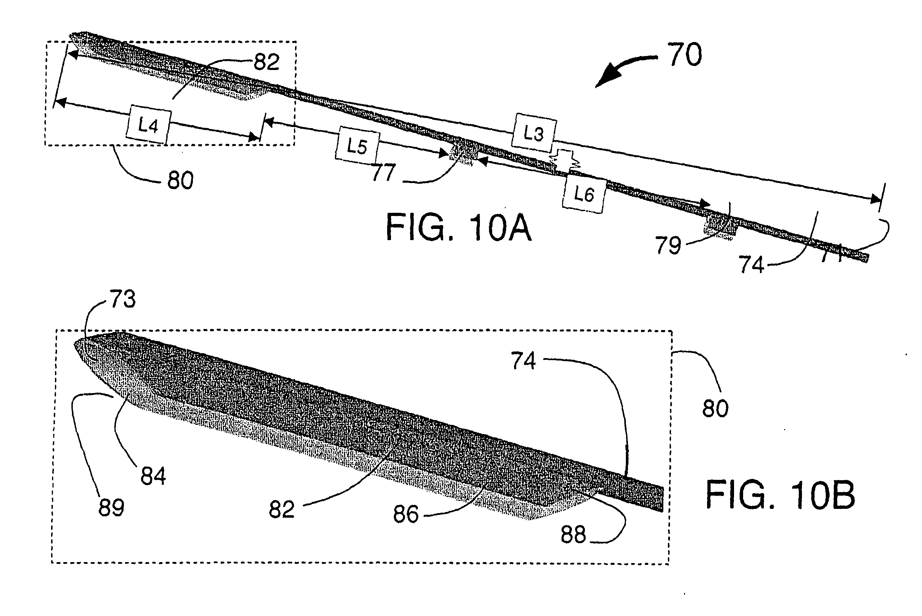

FIG. 10A illustrates a second snap-on embodiment of the present invention rescue catheter 70 having a guidewire tube 74 of length L3 with guidewire lumen 71. The catheter 70 also has a distal end 80 with tapered slit snap-on section 82 having length L4. L3 must be sufficiently long so that the distal end of the catheter 70 can reach the desired point in the human vascular system and is typically between 1 and 2 meters.

L4 is typically between 0.5 and 20 centimeters. The rescue catheter 70 also has additional slit snap-on segments 77 and 79 where the slit snap-on segment 77 lies a distance L5 proximal to the proximal end of the slit snap-on section 82 and the slit snap-on segment 79 lies a distance L6 proximal to the proximal end of the snap-on segment 77. The distances L5 and L6 can be as short as 1 cm or as long as a meter.

Although the rescue catheter 70 has two slit snap on segments 77 and 79, it is envisioned that it could have no slit snap-on segments such as the rescue catheter 60 of FIGs. 9A and 9B or it could have any number from 1 to 100 of the slit snap-on segments. The slit snap-on segments improve the delivery of the rescue catheter 70 by keeping the guidewire tube 74 in close proximity to the shaft 2 of the stent delivery system 1 of FIGs 1 and 2.

It is also envisioned that the slit snap-on segments 77 and 79 can be tapered similar to the distal slit snap-on section 82. While the slit snap-on segments 77 and 79 are shown in FIG. 10A as being relatively short compared to the distal slit snap-on section 82, it is envisioned that the slit snap-on segments 77 and 79 can be of equal or greater length than the distal slit snap-on section 82.

FIG. 10B shows an enlargement of the distal end 80 of the rescue catheter 70 of FIG. 10A. The distal end 80 includes the tapered slit snap-on section 82 with lumen 89 and slit opening 86. The tapered slit snap-on section 82 has a tapered distal end 84 and a tapered proximal end 88 which facilitate penetration through a vascular obstruction and improved delivery over the stent delivery system of FIGs. 1 and 2.

Also shown in FIG. 10B is the tapered distal end 73 of the guidewire tube 74 which facilitates penetration through a vascular obstruction such as an intimal dissection. Unlike the rescue catheters 10 and 50 of FIGs. 3 and 8, the rescue catheter 70 does not require that the fitting at the proximal end of the stent delivery system 1 be cut off. The tapered slit snap-on distal section 82 is designed to be snapped onto the shaft 2 of the stent delivery system 1 of FIGs. 1 and 2. This is done by pressing the shaft 2 through the slit 86 until the shaft 2 snaps into the lumen 89 of the slit snap-on section 82. The rescue catheter can then be advanced over the shaft 2 and beyond until the distal end 73 of the guidewire tube 74 lies beyond the vascular obstruction. A guidewire can than be advanced through the lumen 71 of the guidewire tube 74 until the distal end of the guidewire lies beyond the vascular obstruction. The rescue catheter 70 and stent delivery system 1 can then be removed from the body and the guidewire can be used to perform additional procedures such as stent delivery, to recannalize the vessel. Although both proximal end 88 and distal end 84 of the slit snap-on section 82 are tapered, it is envisioned that only one of the ends 88 and 84 or neither of the ends 88 and 84 need be tapered.

There is also disclosed, but not claimed, a method for repairing an intimal dissection resulting from the placement of a stent that has been delivered from a fixed guidewire stent delivery catheter, the method comprising the following steps:
a) advancing a fixed guidewire stent delivery catheter into a stenosed artery of a human subject;
b) inflating a balloon placed at a distal portion of the fixed guidewire stent delivery catheter so as to deliver a stent mounted onto the balloon into a stenosis of the stenosed artery;
c) deflating the balloon;
d) snapping a slit snap-on section of a rescue catheter having a guidewire tube, the guidewire tube having a distal end, over the shaft of the fixed guidewire stent delivery catheter;
e) advancing the rescue catheter until the guidewire tube's distal end lies distal to the observed intimal dissection;
f) advancing a guidewire through the guidewire tube of the rescue catheter until the guidewire extends beyond the observed intimal dissection;
g) removing the fixed guidewire stent delivery catheter and rescue catheter from the body of the patient.

Optionally, the method further comprises the following step:
pressing the shaft of the fixed guidewire stent delivery catheter through the slit of at least one slit snap-on segment.

Optionally, the method further comprises the following steps:
a) placing a stent delivery catheter over the guidewire so that a stent at a distal portion of the stent delivery catheter is situated at the site of the intimal dissection;
b) delivering the stent into the intimal dissection so as to repair the intimal dissection; and
c) removing the stent delivery catheter and the guidewire from the human subject.

Various other modifications, adaptations, and alternative designs are of course possible in light of the above teachings. Therefore, it should be understood at this time that within the scope of the appended claims, the invention may be practiced otherwise than as specifically described herein.

## Claims

1. A rescue catheter (60; 70) adapted for placement over a fixed guidewire stent delivery catheter (1) after an intimal dissection has been detected following the placement of a stent (5') into an artery of a human body, the rescue catheter comprising:
a guidewire tube (64; 74) having proximal and distal ends (63; 73), and a slit (66; 86),
**characterised in that** the rescue catheter is a rapid exchange rescue catheter (60, 70) and the slit is in a slit snap-on section designed to snap onto the shaft of the fixed guidewire stent delivery catheter (62; 82) fixedly attached near the distal end (63; 73) of the guidewire tube (64; 74).

2. The rescue catheter of claim 1 wherein the distal end (63; 73) of the guidewire tube is tapered.

3. The rescue catheter of claim 1 or claim 2 wherein the slit snap-on section (82) has a tapered distal end (84).

4. The rescue catheter of claim 1, 2 or 3 wherein the slit snap-on section (82) has a tapered proximal end (88).

5. The rescue catheter of any of claims 1 to 4 wherein the slit snap-on section (62; 82) has a length of less than 20 centimeters.

6. The rescue catheter of any of claims 1 to 5 wherein the slit snap-on section (62; 82) has a length of more than 1 centimeter.

7. The rescue catheter of any of claims 1 to 6 further comprising at least one slit snap-on segment (77; 79) fixedly attached to the guidewire tube (74) at a location proximal to the slit snap-on section (82).

8. The rescue catheter of claim 7 wherein the at least one slit snap-on segment (77; 79) has tapered distal end.

9. The rescue catheter of claim 7 or claim 8 wherein the at least one slit snap-on segment (77; 79) has tapered proximal end.

## Patentansprüche

1. Rettungskatheter (60; 70), welcher eingerichtet ist zur Platzierung über einem Stentzuführungskatheter (1) mit festem Führungsdraht, nachdem folgend auf die Platzierung eines Stents (5') in einer Arterie eines menschlichen Körpers eine intimale Dissektion festgestellt wurde, wobei der Rettungskatheter Folgendes umfasst:
eine Führungsdrahtröhre (64; 74) mit einem proximalen und einem distalen Ende (63; 73) und einem Schlitz (66; 86), **dadurch gekennzeichnet, dass** der Rettungskatheter ein Schnellaustauschrettungskatheter (60; 70) ist, und dass sich der Schlitz in einem Schlitzaufschnappabschnitt (62; 82) befindet, welcher eingerichtet ist, um auf den Schaft des Stentzuführungskatheters mit festem Führungsdraht aufzuschnappen, und nahe dem distalen Ende (63; 73) der Führungsdrahtröhre (64; 74) fest angebracht ist.

2. Rettungskatheter nach Anspruch 1, wobei das distale Ende (63; 73) der Führungsdrahtröhre verjüngt ist.

3. Rettungskatheter nach Anspruch 1 oder Anspruch 2, wobei der Schlitzaufschnappabschnitt (82) ein verjüngtes distales Ende (84) aufweist.

4. Rettungskatheter nach Anspruch 1, 2 oder 3, wobei der Schlitzaufschnappabschnitt (82) ein verjüngtes proximales Ende (88) aufweist.

5. Rettungskatheter nach einem der Ansprüche 1 bis 4, wobei der Schlitzaufschnappabschnitt (62; 82) eine Länge von weniger als 20 cm aufweist.

6. Rettungskatheter nach einem der Ansprüche 1 bis 5, wobei der Schlitzaufschnappabschnitt (62; 82) eine Länge von mehr als 1 cm aufweist.

7. Rettungskatheter nach einem der Ansprüche 1 bis 6, welcher weiter zumindest ein Schlitzaufschnappsegment (77; 79) umfasst, welches fest an der Führungsdrahtröhre (74) an einem Ort proximal zu dem Schlitzaufschnappabschnitt (82) angebracht ist.

8. Rettungskatheter nach Anspruch 7, wobei das zumindest eine Schlitzaufschnappsegment (77; 79) ein verjüngtes distales Ende aufweist.

9. Rettungskatheter nach Anspruch 7 oder Anspruch 8, wobei das zumindest eine Schlitzaufschnappsegment (77; 79) ein verjüngtes proximales Ende aufweist.

## Revendications

1. Cathéter à succion (60 ; 70) adapté pour être placé sur un cathéter de pose de stent (1) à fil guide fixe après qu'une dissection intimale a été détectée suite à la mise en place d'un stent (5') dans une artère d'un corps humain, le cathéter à succion comprenant :
un tube de fil guide (64 ; 74) ayant des extrémités proximale et distale (63 ; 73) et une fente (66 ; 86),
**caractérisé en ce que** le cathéter à succion est un cathéter à succion à échange rapide (60, 70) et la fente est dans une section détachable de fente (62 ; 82) conçue pour s'emboîter sur la tige du cathéter de pose de stent à fil guide fixe, fixée de manière fixe à proximité de l'extrémité distale (63 ; 73) du tube de fil guide (64 ; 74).

2. Cathéter à succion selon la revendication 1, dans lequel l'extrémité distale (63 ; 73) du tube de fil guide est progressivement rétrécie.

3. Cathéter à succion selon la revendication 1 ou la revendication 2, dans lequel la section détachable de fente (82) a une extrémité distale (84) progressivement rétrécie.

4. Cathéter à succion selon la revendication 1, 2 ou 3, dans lequel la section détachable de fente (82) a une extrémité proximale (88) progressivement rétrécie.

5. Cathéter à succion selon l'une quelconque des revendications 1 à 4, dans lequel la section détachable de fente (62 ; 82) a une longueur inférieure à 20 centimètres.

6. Cathéter à succion selon l'une quelconque des revendications 1 à 5, dans lequel la section détachable de fente (62 ; 82) a une longueur supérieure à 1 centimètre.

7. Cathéter à succion selon l'une quelconque des revendications 1 à 6, comprenant en outre au moins un segment détachable de fente (77 ; 79) fixé de manière fixe sur le tube de fil guide (74) à un emplacement situé à proximité de la section détachable de fente (82).

8. Cathéter à succion selon la revendication 7, dans lequel le au moins un segment détachable de fente (77 ; 79) a une extrémité distale progressivement rétrécie.

9. Cathéter à succion selon la revendication 7 ou la revendication 8, dans lequel le au moins un segment détachable de fente (77 ; 79) a une extrémité proximale progressivement rétrécie.
